(19) Europäisches Patentamt
European Patent Office
Office européen des brevets

(11) Veröffentlichungsnummer: **0 113 858**
**B1**

(12) # EUROPÄISCHE PATENTSCHRIFT

(45) Veröffentlichungstag der Patentschrift: **17.02.88**

(21) Anmeldenummer: **83112328.6**

(22) Anmeldetag: **08.12.83**

(51) Int. Cl.⁴: **C 07 D 301/32**, C 07 D 301/14, C 07 D 303/16, C 07 D 405/14 // (C07D405/14, 303:36, 251:32)

(54) **Verfahren zur Herstellung und Isolierung von Polyglycidylverbindungen.**

(30) Priorität: **21.12.82 DE 3247255**

(43) Veröffentlichungstag der Anmeldung: **25.07.84 Patentblatt 84/30**

(45) Bekanntmachung des Hinweises auf die Patenterteilung: **17.02.88 Patentblatt 88/7**

(84) Benannte Vertragsstaaten: **DE FR GB IT**

(56) Entgegenhaltungen:
**EP - A - 0 008 111**
**EP - A - 0 008 112**
**EP - A - 0 056 932**
**DE - A - 1 643 852**
**DE - A - 2 747 761**
**DE - A - 2 747 762**
**DE - B - 1 942 557**
**DE - B - 1 953 465**
**DE - B - 2 023 839**
**US - E - 30 945**

**CHEMICAL ABSTRACTS, Vol. 90, No. 10, 5. März 1979, Columbus, Ohio, USA E.M. BLYAKHMAN, A.A. BOTVINKINA "Polyglycidyl esters of cyanuric acid" Seite 30, Spalte 1, Abstract-No. 72 831c**

(73) Patentinhaber: **BAYER AG, Konzernverwaltung RP Patentabteilung, D-5090 Leverkusen 1 Bayerwerk (DE)**

(72) Erfinder: **Rauleder, Gebhard, Dr. c/o Bayer do Brasil S.A., Estrado Boa Esperanza 650, 25150 Belford Roxo (BR)**
Erfinder: **Waldmann, Helmut, Dr., Henry T.v. Böttinger Strasse 15, D-5090 Leverkusen 1 (DE)**
Erfinder: **Bottenbruch, Ludwig, Dr., Wöhlerstrasse 5, D-4150 Krefeld (DE)**
Erfinder: **Traenckner, Hans-Joachim, Dr., Amselweg 2, D-3032 Fallingbostel (DE)**
Erfinder: **Gau, Wolfgang, Dr., Am Eckbusch 39 - 56, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 113 858 B1

**Beschreibung**

Die vorliegende Erfindung betrifft ein Verfahren zur Herstellung von Polyglycidylverbindungen aus Polyallylverbindungen und Percarbonsäuren und die Isolierung der Polyglycidylverbindungen aus den dabei anfallenden Reaktionsgemischen, welches untenstehend näher im einzelnen definiert wird.

Die erfindungsgemäss herstellbaren und isolierbaren Polyglycidylverbindungen sind bekannte Zwischenprodukte und finden beispielsweise als Ausgangsprodukte zur Herstellung von Lacken und Kunststoffen, als Stabilisatoren, für die Herstellung von Giessharzen, für die Herstellung von Freiluftisolatoren, als Weichmacher und als Klebstoffe Verwendung (s. z.B. DE-B-1 082 263 und US-A-2 870 170).

Es ist bekannt, dass man Glycidylester aus Carbonsäuren, Carbonsäureanhydriden und Salzen von Carbonsäuren durch Umsetzung mit Epihalogenhydrin herstellen kann (s. z.B. DE-B-1 165 030, DE-A-1 643 777, US-A-2 448 602 und DE-A-2 023 148). Ein grundsätzlicher Nachteil dieser Verfahren besteht darin, dass dabei salzhaltige umweltbelastende Abwässer gebildet werden.

In der GB-A-735 001 wird die Herstellung von Glycidylestern durch Umsetzen von Glycid mit Polycarbonsäurechloriden beschrieben. Dieses Verfahren hat den grossen Nachteil, dass die Ausgangsmaterialien instabil und sehr reaktionsfähig sind, so dass bei der Herstellung, der Lagerung und der Verwendung dieser Verbindungen äusserste Vorsicht erforderlich ist (s. DE-A-2 032 148, S. 1, Zeile 6).

Eine Möglichkeit, Olefine in Epoxide umzuwandeln unter Umgehung dieser Nachteile, besteht in der Anwendung der «Prileschajew-Reaktion» (vgl. N. Prileschajew, Ber. dtsch. chem. Ges. 42, 4811 (1909)). Bei dieser Reaktion handelt es sich um einen elektrophilen Angriff einer Percarbonsäure auf ein Olefin (vgl. K.D. Bingham, G.D. Meakins, G.H. Witham, Chem. Comm. 1966, S. 445 und 446). Aus diesem Grunde nimmt die Reaktivität des Olefins mit fallender Nukleophilie der Doppelbindung ab. Deshalb erschweren elektronegative Substituenten die Epoxidation (vgl. S.N. Lewis in R.L. Augustin, «Oxidation» Vol. I, S. 227, Z. 9–13, Marcel Dekker, New York (1969)).

Elektronenanziehende Gruppen, wie z.B. Carboxyl, Carbonyl usw. vermindern die Reaktionsgeschwindigkeit enorm (vgl. H. Batzer und E. Nikles, Chimia, Vol. 16, S. 62 (1962)). Insbesondere lassen sich daher Allylverbindungen nicht ohne weiteres mit Percarbonsäuren epoxidieren (vgl. DE-A-2 023 148). Infolge des geringen Reaktionsfähigkeit ihrer Doppelbindung sind hohe Temperaturen und lange Reaktionszeiten erforderlich, was zur Bildung von unerwünschten Nebenprodukten, wie Dihydroxy- und Hydroxyacyloxy-Derivaten der Ausgangsprodukte Anlass gibt (vgl. S.N. Lewis in R.L. Augustin, «Oxidation» Vol. I, S. 233, Z. 6–11, Marcel Dekker, New York (1969)).

In der GP-A-862 588 wird vorgeschlagen, den Allylester der Terephthalsäure mit 50%igem Wasserstoffperoxid in Gegenwart eines Kationenaustauschers, der Essigsäure adsorbiert enthält, zu epoxidieren (vgl. S. 2, Zeile 15). Die Verwendung eines Kationenaustauschers als Katalysator hat jedoch den Nachteil, dass er vom Reaktionsgemisch angegriffen wird, so dass eine wiederholte Verwendung nur begrenzt möglich ist (vgl. DE-B-1 082 263, Seite 1, Zeile 40).

In der DE-B-1 082 263 wird im Beispiel 9, Seite 8, Zeile 40, die Epoxidation von Terephthalsäurediallylester mit «in situ» hergestellter Peressigsäure in Gegenwart von Aluminiumoxid beschrieben. Für eine technische Anwendung stellt jedoch die grosse Menge an festem Katalysator im Reaktionsgemisch (70 g Aluminiumoxid auf 1 Mol Terephthalsäurediallylester) einen besonderen Nachteil dar. Der Katalysator muss nach beendeter Reaktion vom Reaktionsgemisch abgetrennt, gereinigt und regeneriert werden, wobei die Häufigkeit seiner Wiederverwendung sehr begrenzt ist.

In zwei weiteren Patentschriften (US-A-3 155 638 und FR-B-1 394 195) wird vorgeschlagen, zur Epoxidation von ethylenisch ungesättigten Carbonsäureestern Monoperphthalsäure zu verwenden. Ein Nachteil dieser Methode ist jedoch die technisch aufwendige Rückgewinnung der Phthalsäure aus dem Reaktionsgemisch. So wird in der US-A-3 155 638 im Beispiel 11, Seiten 8 und 9, beschrieben, dass man nach der Umsetzung von Phthalsäurediallylester mit Phthalsäureanhydrid und Wasserstoffperoxid die abgeschiedene Phthalsäure abfiltriert. Der im Filtrat verbleibende Rest muss durch wiederholtes Waschen mit Wasser und Natronlauge rückgewonnen werden und daran anschliessend müssen zur Wiedergewinnung der in den wässrigen Phasen enthaltenen Phthalsäure diese wieder angesäuert werden, was zur Bildung unerwünschter Salzabfälle führt. Im Beispiel 12 dieser US-PS wird Maleinsäurediallylester mit Phthalsäureanhydrid und Wasserstoffperoxid umgesetzt. Nach der gleichen Aufarbeitungsprozedur wie oben beschrieben resultiert Allylglycidylmaleat in einer Ausbeute von nur 24% der Theorie. Diglycidylmaleat wurde nicht erhalten.

Auch die Verwendung von m-Chlorperbenzoesäure zur Epoxidation der Allylester von Polycarbonsäuren, wie sie in der Literatur vorgeschlagen wurde (S.R. Sandler und F.R. Berg, J. Chem. and Eng. Data, Vol. 11, S. 447 und 448 (1966)), ist für eine technische Anwendung nicht geeignet, da die vorgeschlagenen Reaktionsbedingungen von 3 bis 5 °C, die Verwendung von kostspieliger Kühlsole notwendig macht und die angegebene Reaktionszeit von 3 Tagen technisch nicht akzeptabel ist. Trotz dieser aufwendigen Reaktionsbedingungen wurde beispielsweise Diglycidylterephthalat nur in einer Ausbeute von 28% der Theorie erhalten (vgl. Seite 448, Zeile 13).

In der US-A-2 761 870 wird in den Beispielen 1 bis 5 und Beispiel 8 die Epoxidierung von Crotylestern von Carbonsäuren mit 45%-iger Peressigsäure beschrieben. Auch hier sind die Reaktionsbedingungen, das Reaktionsgemisch muss 2 Tage

bei 0 bis 25 °C stehen, für eine technische Anwendung (Verwendung von Kühlsole, lange Reaktionszeit) äusserst aufwendig. Nach der Reaktion wird die Essigsäure durch Waschen mit 20%iger Natronlauge aus dem Reaktionsgemisch entfernt. Will man die Essigsäure wiedergewinnen, muss die wässrige Phase wieder angesäuert werden, was zur Bildung von umweltbelastenden Salzabfällen führt.

In der US-A-3 155 638, Beispiel 13, wird nach der Umsetzung von Fumarsäurediallylester mit Phthalsäureanhydrid und Wasserstoffperoxid die Phthalsäure abfiltriert. Der im Filtrat verbleibende Rest muss durch wiederholtes Waschen mit Natronlauge und Wasser zurückgewonnen werden und anschliessend müssen zur Wiedergewinnung der in den wässrigen Phasen enthaltenden Phthalsäure diese wieder angesäuert werden, was zur Bildung unerwünschter Salzabfälle führt. Bei diesem Verfahren wird nur Allylglycidylfumarat und dieses nur in einer Ausbeute von 34% der Theorie erhalten.

Über die Umsetzung von Maleinsäurediallylester mit Peressigsäure wird in J. Am. Chem. Soc. 81, S. 3354 (1959) berichtet. Trotz 4-fachem Überschuss an Ester betrug die Reaktionszeit 11,5 Stunden bei 50 °C. Allylglycidylmaleat wurde in einer Ausbeute von nur 71% erhalten. Die Herstellung von Diglycidylmaleat ist nicht erwähnt.

In der US-A-2 783 250, Beispiel 2, wird die Umsetzung von Tetrahydrophthalsäurediallylester mit Peressigsäure in Chloroform beschrieben. Nach einer Reaktionsdauer von 10 Stunden bei 0 bis 10 °C wurde nach dem Abtrennen der Essigsäure und des Lösungsmittels ein hochviskoser Rückstand erhalten, der als 7-Oxabicyclo-(4.1.0)-heptan-3,4-carbonsäureallylester identifiziert wurde.

In der US-A-2 870 170, Beispiel 1, wird die Umsetzung von Tetrahydrophthalsäurediallylester mit Peressigsäure in Chloroform beschrieben. Nach einer Reaktionszeit von 2 Tagen bei 0 bis 10 °C wurde nach Abtrennen der Essigsäure und des Lösungsmittels ein Rückstand erhalten, der als 7-Oxabicyclo-(4.1.0)-heptan-3,4-dicarbonsäureallyl-glycidylester identifiziert wurde. Die Ausbeute betrug 50% der Theorie.

In der US-A-2 870 170 ist ein Extraktionsverfahren beschrieben, bei dem die Gesamtheit der epoxidierten Produkte aus dem Reaktionsgemisch abgetrennt wird. Eine Trennung der verschiedenen epoxidierten Produkte voneinander wird nicht beschrieben.

Gemäss der US-A-2 783 250 werden nur im Säureteil epoxidierte Ester hergestellt. Das Entstehen von Polyglycidylverbindungen wird ausdrücklich unterdrückt (siehe Spalte 8, Zeilen 60–64). Im Verfahren der US-A-3 155 638 wird eine unselektive Abtrennung von epoxidierten Produkten aus einem Reaktionsgemisch durch Filtration entstandener Dicarbonsäure, Auswaschen mit Alkali und Einengung unter vermindertem Druck durchgeführt.

Ähnlich wie in der US-A-2 870 170 werden in der DE-A-1 643 852 Epoxyverbindungen (keine Polyglycidylverbindungen) von Säuren getrennt, hier allerdings auf destillativem Wege.

Die DE-A-2 747 762 betrifft die Herstellung von Percarbonsäure, deren Umsetzung mit einem Alken oder Alkenderivat und die Aufarbeitung des Reaktionsgemisches durch Destillation. Eine selektive Isolierung von Polyglycidylverbindungen wird nicht beschrieben.

Schliesslich betreffen die EP-OS'en 8111 und 8112 die Herstellung von Polyglycidylverbindungen, nicht jedoch die Isolierung der jeweils vollständig epoxidierten Verbindungen aus der hydrophilen Phase in einer gleichzeitigen Extraktion mit einer lipophilen und einer hydrophilen Phase.

Es wurde nun ein Verfahren zur Herstellung von Polyglycidylverbindungen gefunden, bei dem man eine Polyallylverbindung der allgemeinen Formel

$$R-(\overset{O}{\overset{\|}{C}}-O-CH_2-CH=CH_2)_n$$

worin

R für einen, gegebenenfalls substituierten, gesättigten, aliphatischen Rest mit 1 bis 10 C-Atomen, für die Gruppe $-CH=CH-$, einen, gegebenenfalls substituierten, gesättigten oder ungesättigten cycloaliphatischen Rest mit 5 bis 12 C-Atomen, für einen, gegebenenfalls substituierten, Benzolrest oder für einen, gegebenenfalls substituierten, Naphthalinrest und

n für 2, 3 oder 4 steht

oder eine Triallylverbindung von Cyanursäure, Isocyanursäure oder Urazol bei 30 bis 100 °C mit 0,6 bis 1,1 Mol einer 3 oder 4 Kohlenstoffatome enthaltenden Percarbonsäure, bezogen auf 1 Mol zu epoxidierender Doppelbindung, umsetzt und anschliessend durch Destillation die aus der Percarbonsäure entstandene Carbonsäure abtrennt, das dadurch gekennzeichnet ist, dass man das verbleibende Gemisch durch gleichzeitige Extraktion mit einer lipophilen und einer hydrophilen Phase auftrennt, anschliessend getrennt die beladene lipophile und die beladene hydrophile Phase in an sich bekannter Weise aufarbeitet und als Rückstand aus der Aufarbeitung der beladenen hydrophilen Phase die Polyglycidylverbindung erhält.

Wenn als Polyallylverbindung eine Verbindung der vorstehenden allgemeinen Formel eingesetzt wird, so kann der Rest R, sofern er substituiert ist, als Substituenten beispielsweise $C_1$- bis $C_4$-Alkyl- und/oder $C_1$- bis $C_4$-Alkoxygruppen aufweisen. In der vorstehend angegebenen allgemeinen Formel steht n vorzugsweise für 2 oder 3.

Zum Einsatz in das erfindungsgemässe Verfahren geeignete Polyallylverbindungen sind beispielsweise die Polyallylester von

Hexahydrophthalsäure, Hexahydroisophthalsäure,
Hexahydroterephthalsäure,
3-Methylhexahydrophthalsäure,
3,5-Dimethylhexahydrophthalsäure,
hydrierten Benzoltri- und -tetracarbonsäuren,
Tetrahydrophthalsäure, 3-Methylphthalsäure,

3,5-Dimethylphthalsäure, 3-Methoxyphthalsäure, Terephthalsäure, Benzoltricarbonsäuren, Benzoltetracarbonsäuren, Naphthalindicarbonsäuren, Malonsäure, Bernsteinsäure, Glutarsäure, Adipinsäure, Pimelinsäure, Suberinsäure, Decandicarbonsäure, Maleinsäure, Fumarsäure und die Trisallylverbindungen von Cyanursäure, Isocyanursäure und Urazol.

Besonders geeignet zur Umsetzung mit Percarbonsäuren nach dem erfindungsgemässen Verfahren sind die Diallylester von 3-Methyl-hexahydrophthalsäure, 3-Methoxyhexahydrophthalsäure, Hexahydrophthalsäure, Hexahydroisophthalsäure, Hexahydroterephthalsäure, Methylphthalsäure, Phthalsäure, Terephthalsäure, Tetrahydrophthalsäure, Malonsäure, Bernsteinsäure, Glutarsäure, Adipinsäure, Maleinsäure, Fumarsäure und die Trisallylverbindungen von Isocyanursäure und Urazol.

Ganz besonders geeignet zum Einsatz in das erfindungsgemässe Verfahren sind die Diallylester von Hexahydrophthalsäure, Hexahydroterephthalsäure, Tetrahydrophthalsäure, Phthalsäure, Malonsäure, Bernsteinsäure, Glutarsäure, Adipinsäure, Maleinsäure, Fumarsäure und die Trisallylverbindungen von Isocyanursäure und Urazol.

Die in das erfindungsgemässe Verfahren einsetzbaren Polyallylester von Polycarbonsäuren können in an sich bekannter Weise erhalten werden, beispielsweise durch Veresterung der entsprechenden Säureanhydride oder Säuren mit Allylalkohol. Die Trisallylverbindungen von Cyanursäure, Isocyanursäure und Urazol können beispielsweise durch Umsetzung dieser Verbindungen mit Allylchlorid erhalten werden.

Die Umsetzung mit der Percarbonsäure wird bevorzugt in Gegenwart organischer Lösungsmittel durchgeführt. Als organische Lösungsmittel können die verschiedensten unsubstituierten und substituierten Kohlenwasserstoffe verwendet werden, die unter Reaktionsbedingungen flüssig sind und unerwünschte Nebenreaktionen nicht oder nur in ganz untergeordnetem Masse eingehen. Als Kohlenwasserstoffe können z.B. verwendet werden: Aliphatische und cycloaliphatische Kohlenwasserstoffe wie Hexan, Heptan, Octan, 2-Ethyl-hexan, Decan, Dodecan, Cyclohexan, Methylcyclopentan und Petrolether, weiterhin aromatische Kohlenwasserstoffe wie Benzol, Nitrobenzol, Toluol, Ethylbenzol, Cumol, Diisopropylbenzol, Xylol Chlorbenzol und Dichlorbenzol, weiterhin sauerstoffhaltige Kohlenwasserstoffe wie Diethylether, Diisopropylether, Dibutylether, Tetrahydrofuran, Dioxan, Essigsäureethylester, Essigsäuremethylester, Essigsäurepropylester, Essigsäurebutylester, Propionsäuremethylester, Propionsäureethylester, Propionsäurepropylester, Buttersäuremethylester, Buttersäureethylester, Buttersäurepropylester, Buttersäurebutylester, Benzoesäuremethylester und Benzoesäureethylester, weiterhin chlorierte Kohlenwasserstoffe wie Methylenchlorid, Chloroform, Tetrachlorkohlenstoff, 1-Chlorethan, 1,2-Dichlorethan, 1,1-Dichlorethan, 1,1,2,2-Tetrachlorethan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, Chlorbenzol, Heptylchlorid, 1,2-Dichlorheptan, 1,2,3,4-Tetrachlorheptan, Cycloheptylchlorid, Octylchlorid, 1,2-Dichloroctan, 1,2,3,4-Tetrachloroctan und Cyclooctylchlorid.

Bevorzugte Lösungsmittel sind von den chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol, Chlorbenzol und Dichlorbenzol, von den aliphatischen und cycloaliphatischen Kohlenwasserstoffen 2-Ethylhexan, Cyclohexan und Methylcyclopentan und von den sauerstoffhaltigen Kohlenwasserstoffen Tetrahydrofuran, Propionsäureethylester und Benzoesäureethylester.

Besonders bevorzugte Lösungsmittel sind von den chlorierten Kohlenwasserstoffen 1,2-Dichlorpropan und Tetrachlorkohlenstoff, von den aromatischen Kohlenwasserstoffen Benzol und Dichlorbenzol, von den aliphatischen und cycloaliphatischen Kohlenwasserstoffen Cyclohexan und von den sauerstoffhaltigen Kohlenwasserstoffen Propionsäureethylester.

Verwendet werden können auch Gemische der verschiedenen oben angegebenen organischen Lösungsmittel.

In das Verfahren der vorliegenden Erfindung einsetzbare Percarbonsäuren sind Perpropionsäure, Perbuttersäure und Perisobuttersäure. Bevorzugt verwendet werden Perpropionsäure und Perisobuttersäure, besonders bevorzugt ist Perpropionsäure.

Diese Percarbonsäuren, gelöst in einem der genannten organischen Lösungsmittel, können z.B. nach dem in der DE-A-2 262 970 beschriebenen Verfahren hergestellt werden, bei dem wässriges Wasserstoffperoxid mit der entsprechenden Carbonsäure in Gegenwart von Schwefelsäure

umgesetzt und anschliessend die entstandene Percarbonsäure mit einem organischen Lösungsmittel aus dem Reaktionsgemisch extrahiert wird. Gegebenenfalls kann die so erhaltene Percarbonsäurelösung in dem organischen Lösungsmittel noch weiter gereinigt werden, insbesondere um den Gehalt an Wasser, Wasserstoffperoxid und Schwefelsäure zu erniedrigen.

Im allgemeinen verwendet man die Percarbonsäure in Form einer Lösung in einem organischen Lösungsmittel. Derartige Percarbonsäurelösungen können beispielsweise 10 bis 30 Gew.-% der jeweiligen Percarbonsäure, bezogen auf die Lösung, enthalten. Die Polyallylverbindungen können als solche oder ebenfalls gelöst in einem oder mehreren der vorstehend genannten Lösungsmittel eingesetzt werden, wobei beliebig konzentrierte Lösungen der Polyallylverbindungen zum Einsatz gelangen können. Vorzugsweise werden die Polyallylverbindungen als solche eingesetzt und organische Lösungsmittel nur in Form der Percarbonsäurelösung zugegeben.

Pro Mol zu epoxidierender Doppelbindung werden 0,6 bis 1,1 Mol Percarbonsäure eingesetzt. Bevorzugt werden pro Mol zu epoxidierender Doppelbindung 0,7 bis 1 Mol Percarbonsäure eingesetzt, besonders bevorzugt 0,75 bis 0,9 Mol Percarbonsäure pro Mol zu epoxidierender Doppelbindung.

Der Wassergehalt der verwendeten Percarbonsäurelösung soll im allgemeinen möglichst niedrig sein. Wassermengen bis 10 Gew.-% in der Percarbonsäurelösung sind im allgemeinen nicht störend. Geeignet ist beispielsweise eine Percarbonsäurelösung mit einem Wassergehalt von bis zu 2 Gew.-%. Vorzugsweise verwendet man eine Percarbonsäurelösung, die weniger als 1 Gew.-% Wasser enthält. Besonders bevorzugt ist ein Wassergehalt von weniger als 0,1 Gew.-%.

Der Wasserstoffperoxidgehalt der verwendeten Percarbonsäurelösung in einem der obengenannten organischen Lösungsmittel soll im allgemeinen möglichst niedrig sein. Er kann beispielsweise bis zu 1 Gew.-%, bezogen auf die Percarbonsäurelösung, betragen. Vorteilhaft arbeitet man mit einem Gehalt von weniger als 0,5 Gew.-%. Besonders vorteilhaft ist es, die Umsetzung mit einer Percarbonsäurelösung durchzuführen, die einen Wasserstoffperoxidgehalt unterhalb 0,2 Gew.-% aufweist.

Der Mineralsäuregehalt der verwendeten Percarbonsäurelösung soll möglichst niedrig sein. Vorteilhaft ist es, die Reaktion mit einer Percarbonsäurelösung durchzuführen, die einen Mineralsäuregehalt unterhalb 50 ppm besitzt. Besonders vorteilhaft ist ein Mineralsäuregehalt von weniger als 10 ppm.

Die erfindungsgemässe Umsetzung wird in dem Temperaturbereich von 30 bis 100 °C durchgeführt. Bevorzugt arbeitet man bei 40 bis 80 °C, besonders bevorzugt bei 50 bis 75 °C. In Sonderfällen können die angegebenen Temperaturen auch unter- oder überschritten werden.

Neben der Arbeitsweise unter isothermen Bedingungen, d.h. Einhaltung einer einheitlichen Temperatur im gesamten Reaktionsgemisch, kann man die erfindungsgemässe Umsetzung auch unter Ausbildung eines sog. Temperaturgradienten durchführen, der im allgemeinen mit fortschreitender Reaktion zunimmt. Man kann aber auch die Reaktion so führen, dass sich mit dem Fortschreiten der Umsetzung ein Gradient fallender Temperatur ausbildet.

Die erfindungsgemässe Umsetzung kann bei den verschiedensten Drucken durchgeführt werden. Im allgemeinen arbeitet man bei Normaldruck. Die Umsetzung kann jedoch auch bei Unter- oder Überdruck durchgeführt werden.

Die Durchführung der erfindungsgemässen Umsetzung kann diskontinuierlich oder kontinuierlich in den für Umsetzungen dieser Art üblichen Vorrichtungen, wie Rührwerkskessel, Siedereaktoren, Röhrenreaktoren, Schlaufenreaktoren oder Schleifenreaktoren, erfolgen.

Als Werkstoffe für die Reaktionsapparate zur Durchführung der erfindungsgemässen Umsetzung können beispielsweise Glas, Edelstähle oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die Schwermetallionen durch Komplexbildung inaktivieren können. Bekannte Substanzen solcher Art sind beispielsweise Gluconsäure, Ethylendiamintetraessigsäure, Natriumsilikat, Natriumpyrophosphat, Natriumhexametaphosphat, Dinatriumdimethylpyrophosphat oder $Na_2$ (2-Ethylhexyl)$_5$ $(P_3O_{10})_2$ (vgl. DE-B-1 056 596, Spalte 4, Zeile 60 ff).

Die Reaktionswärme kann durch innen- oder aussenliegende Kühler abgeführt werden. Zur Ableitung der Reaktionswärme kann die Umsetzung auch unter Rückfluss (z.B. in Siedereaktoren) durchgeführt werden.

Die Polyallylverbindung und die Percarbonsäure können auf beliebige Weise zusammengebracht werden. Beispielsweise kann man beide Komponenten gleichzeitig oder nacheinander in beliebiger Reihenfolge in das Reaktionsgefäss einbringen. Bei diskontinuierlicher Arbeitsweise wird vorzugsweise die Polyallylverbindung vorgelegt und die Percarbonsäurelösung zugegeben. Die Reaktionstemperatur kann dabei vor oder nach der Zugabe der Percarbonsäure eingestellt werden. Man kann auch die beiden Komponenten bei Raumtemperatur gleichzeitig in das Reaktionsgefäss eingeben und dann die Reaktionstemperatur einstellen. Bei kontinuierlicher Arbeitsweise kann man die beiden Komponenten gemeinsam oder getrennt dem Reaktor zuführen. Bei Verwendung mehrerer Reaktoren, die beispielsweise als Kaskade hintereinandergeschaltet sein können, kann es vorteilhaft sein, die Polyallylverbindungen nur in den ersten Reaktor einzubringen. Man kann die Polyallylverbindung jedoch auch in mehrere Reaktoren einspeisen.

Es kann weiterhin von Vorteil sein, die benötigte Menge an Percarbonsäure chargenweise zuzugeben. Dabei kann es auch von Vorteil sein, nach Zugabe einzelner Chargen die aus der Percarbonsäure entstandene Carbonsäure aus dem Reaktionsgemisch zu entfernen.

Die nach Durchführung der erfindungsgemässen Umsetzung erhaltenen Reaktionsgemische enthalten im allgemeinen das verwendete organische Lösungsmittel, die aus der Percarbonsäure entstandene Carbonsäure, nicht-umgesetzte Polyallylverbindung, partiell epoxidierte Polyallylverbindung, vollständig epoxidierte Polyallylverbindung (= Polyglycidylverbindung) sowie gegebenenfalls geringe Mengen an hochsiedenden Nebenprodukten.

Zur Aufarbeitung wird aus dem Reaktionsgemisch zunächst die aus der Percarbonsäure entstandene Carbonsäure durch Destillation abgetrennt. Vorzugsweise führt man diese Destillation in Gegenwart eines Lösungsmittels durch, das zwischen der aus der Percarbonsäure entstandenen Carbonsäure und der Polyallylverbindung siedet, wobei man dann ein Kopfprodukt abtrennt, welches Lösungsmittel und die Carbonsäure enthält und ein Sumpfprodukt abtrennt, das unumgesetzte Polyallylverbindung, partiell epoxidierte Polyallylverbindung, Polyglycidylverbindung und restliches Lösungsmittel enthält.

Sofern für die Percarbonsäure ein Lösungsmittel verwendet wurde, das einen geeigneten Siedepunkt aufweist, kann die gesonderte Zugabe eines Lösungsmittels unterbleiben. Sonst ist es vorteilhaft vor der Destillation ein weiteres Lösungsmittel zuzufügen, das zwischen der aus der Percarbonsäure entstandenen Carbonsäure und der Polyallylverbindung siedet, beispielsweise in einer Menge von 20 bis 100 Gew.-%, bezogen auf das Reaktionsgemisch. Vorzugsweise beträgt diese Menge 30 bis 80 Gew.-%. Wenn ein weiteres Lösungsmittel eingesetzt wird, geht gegebenenfalls mit der Percarbonsäure eingebrachtes Lösungsmittel im allgemeinen vollständig in das Kopfprodukt und das Sumpfprodukt enthält dann nur Reste des weiteren Lösungsmittels.

Diese Destillation kann beispielsweise bei einem Druck im Bereich von 100 bis 400 mbar und einer Sumpftemperatur im Bereich 90 bis 140 °C durchgeführt werden.

Durch die bevorzugte Arbeitsweise in Gegenwart eines Hilfslösungsmittels kann man im allgemeinen eine vollständige Abtrennung der Carbonsäure bei relativ niedrigen Sumpftemperaturen erreichen, was hinsichtlich der Minimierung der Bildung von Nebenprodukten aus der Carbonsäure und den epoxidierten Bestandteilen des Reaktionsgemisches von Vorteil ist.

Geeignete Hilfslösungsmittel sind beispielsweise Dichlorbenzol, Benzoesäureethylester, Diethylenglykoldiethylether und Diethylenglykoldimethylether. Vorzugsweise wird hierfür Dichlorbenzol verwendet.

Es kann vorteilhaft sein, dem Reaktionsgemisch vor oder während dieser destillativen Aufarbeitung Stabilisatoren zuzusetzen, welche die Bildung von Hochsiedern und Polymerisaten verhindern können.

Zur Isolierung der Polyglycidylverbindung aus dem Carbonsäure- und zumindest weitgehend Lösungsmittel-freien Reaktionsgemisch wird das nach der Destillation verbleibende Gemisch durch gleichzeitige Extraktion mit einer lipophilen und einer hydrophilen Phase aufgetrennt. Dabei verwendet man vorzugsweise eine lipophile Phase aus Cycloalkanen, wie Cyclohexan oder Methylcyclopentan oder aus Mischungen aus 60 bis 90 Gew.-% Alkanen mit 5 bis 12 C-Atomen oder 60 bis 90 Gew.-% Cycloalkanen und jeweils 10 bis 40 Gew.-% Benzol, Toluol, Xylol, Chlorbenzol, Dichlorbenzol, Chloroform, Butylacetat oder Tetrahydrofuran und eine hydrophile Phase aus Ethylenglykol, Diethylenglykol, Sulfolan oder N-Methylpyrrolidon oder aus Mischungen aus 30 bis 80 Gew.-% Wasser und 20 bis 70 Gew.-% Methanol, Ethanol oder Isopropanol oder aus Mischungen aus 10 bis 25 Gew.-% Wasser und 75 bis 90 Gew.-% Ethylenglykol, Diethylenglykol oder Sulfolan oder aus Mischungen aus 10 bis 80 Gew.-% Wasser und 20 bis 90 Gew.-% N-Methylpyrrolidon oder aus Mischungen aus 50 bis 90 Gew.-% Ethylenglykol und 10 bis 50 Gew.-% Methanol, Ethanol oder Isopropanol.

Besonders bevorzugt verwendet man eine lipophile Phase aus Cyclohexan oder Methylcyclohexan oder aus Mischungen aus 60 bis 90 Gew.-% Alkanen mit 5 bis 12 C-Atomen oder 60 bis 90 Gew.-% Cycloalkanen und jeweils 10 bis 40 Gew.-% Benzol, Dichlorbenzol, Chloroform oder Tetrahydrofuran und eine hydrophile Phase aus Ethylenglykol, Diethylenglykol, Sulfolan oder N-Methylpyrrolidon oder aus Mischungen aus 30 bis 50 Gew.-% Wasser und 50 bis 70 Gew.-% Methanol, Ethanol oder Isopropanol oder aus Mischungen aus 75 bis 90 Gew.-% Sulfolan und 10 bis 25 Gew.-% Wasser oder aus Mischungen aus 50 bis 80 Gew.-% N-Methylpyrrolidon und 20 bis 50 Gew.-% Wasser.

Ganz besonders bevorzugt für die Bildung der hydrophilen Phase sind im allgemeinen Mischungen bestehend aus 30 bis 50 Gew.-% Wasser und 50 bis 70 Gew.-% Methanol oder N-Methylpyrrolidon. In Einzelfällen kann der Wassergehalt hierbei bis zu 80 Gew.-% betragen, so beispielsweise bei der Trennung der Reaktionskomponenten aus der Umsetzung von Percarbonsäure mit Trisallylurazol. Ganz besonders bevorzugt für die Bildung der lipophilen Phase sind Cyclohexan oder Mischungen bestehend aus 60 bis 90 Gew.-% Alkanen mit 5 bis 12 C-Atomen oder 60 bis 90 Gew.-% Cyclohexan und jeweils 10 bis 40 Gew.-% Benzol oder Dichlorbenzol.

Die Durchführung der Extraktion kann in üblichen Extraktoren, wie Mischer-Scheider, Siebbodenextraktoren, pulsierten Siebbodenextraktoren oder Drehscheibenextraktoren erfolgen.

Die zur Extraktion notwendigen Mengen an hydrophiler und lipophiler Phase sind abhängig von der Löslichkeit der zu trennenden Komponenten in ihnen. Die Menge an hydrophiler und lipophiler Phase ist vorzugsweise möglichst gering, d.h. gerade ausreichend, um das zu trennende Gemisch vollständig zu lösen. Dabei beträgt das Gewichtsverhältnis von lipophiler zu hydrophiler Phase im

allgemeinen 1:1 bis 5 : 1, vorzugsweise 2 : 1 bis 4 : 1.

Bei den ganz besonders bevorzugt für die Bildung der hydrophilen Phase einzusetzenden Gemischen richtet sich der Wassergehalt nach der Löslichkeit der Polyglycidylverbindung in dieser Phase. Bei relativ gut löslichen Polyglycidylverbindungen, beispielsweise denen, die in Gemischen aus der Umsetzung von Diallylestern der Malon- oder Maleinsäure mit Percarbonsäuren enthalten sind, beträgt der Wassergehalt der hydrophilen Phase vorzugsweise 40 bis 50 Gew.-%. Dadurch ist es möglich, die Menge der lipophilen Phase gering zu halten. Sie kann noch weiter reduziert werden, wenn die lipophile Phase Aromaten enthält.

Bei weniger gut löslichen Polyglycidylverbindungen wird vorzugsweise der Anteil an Methanol bzw. N-Methylpyrrolidon in der hydrophilen Phase möglichst hoch gewählt, ebenso der Anteil an Aromaten in der lipophilen Phase, unter Beachtung, dass eine Mischungslücke zwischen lipophiler und hydrophiler Phase und ein ausreichend hoher Trennfaktor bestehen bleiben.

Wenn man in Vorversuchen für die extraktive Trennung eines bestimmten Gemisches die optimale Zusammensetzung der hydrophilen und lipophilen Phase ermitteln will, d.h. die beste Kombination eines hohen Trennfaktors mit geringem Volumen der Phasen, so braucht man nur die Trennung der vollständig epoxidierten Polyallylverbindung (= Polyglycidylverbindung) von der partiell epoxidierten Polyallylverbindung, die noch eine Allylgruppe enthält, zu betrachten. Die weniger expodierten und nicht-umgesetzten Polyallylverbindungen müssen untereinander nicht aufgetrennt werden und sind in ihrem Extraktionsverhalten den partiell epoxidierten Polyallylverbindungen, die noch eine Allylgruppe enthalten, ähnlich.

Die Extraktion wird im allgemeinen bei Normaltemperatur durchgeführt. Man kann die Extraktion jedoch auch bei erniedrigter oder erhöhter Temperatur durchführen, beispielsweise bei Temperaturen im Bereich von 10 bis 60 °C.

Die Aufarbeitung der beladenen lipophilen und der beladenen hydrophilen Phase erfolgt getrennt und in an sich bekannter Weise. Dabei gewinnt man aus der beladenen lipophilen und der beladenen hydrophilen Phase durch getrennte Destillation die unbeladenen Phasen wieder zurück und führt sie in die Extraktion zurück. Diese Destillationen können beispielsweise bei einem Druck im Bereich von 10 bis 150 mbar und bei einer Sumpftemperatur im Bereich von 90 bis 140 °C durchgeführt werden. Der dabei aus der lipophilen Phase anfallende Rückstand, der unumgesetzte Polyallylverbindung, partiell epoxidierte Polyallylverbindungen und gegebenenfalls geringe Mengen an vollständig epoxidierten Polyallylverbindungen (=Polyglycidylverbindungen) enthält, kann, nach Zusatz frischer Polyallylverbindung, in die Umsetzung mit der Percarbonsäure zurückgeführt werden. Die Zugabe frischer Polyallylverbindung kann auch direkt in das Reaktionsgefäss erfolgen. In besonderen Fällen, beispielsweise wenn nach der Destillation des Reaktionsgemisches zur Abtrennung der Carbonsäure ein fester Rückstand anfällt, kann es vorteilhaft sein, die frische Polyallylverbindung bereits dem Sumpfprodukt dieser Destillation zuzufügen und das Sumpfprodukt dieser Destillation zusammen mit der frischen Polyallylverbindung der Extraktion zuzuführen.

Als Rückstand aus der Destillation der beladenen hydrophilen Phase erhält man die gewünschten Polyglycidylverbindungen. Erfindungsgemäss gelingt es, diese in hohen Ausbeuten und Reinheiten zu isolieren.

Das erfindungsgemässe Verfahren hat eine Reihe von Vorteilen. Gegenüber den eingangs beschriebenen Anwendungen der Prileschajew-Reaktion besteht ein besonderer Vorteil des erfindungsgemässen Verfahrens darin, dass die aus der Percarbonsäure entstehende Carbonsäure durch solche Massnahmen aus dem Reaktionsgemisch entfernt werden kann, bei denen keine umweltbelastenden, salzhaltigen Abwässer anfallen. Die Verwendung eines Katalysators, beispielsweise eines Ionenaustauschers oder Aluminiumoxid, während der Reaktion, ist nicht notwendig. Gegenüber den zitierten Publikationen ist es nach dem erfindungsgemässen Verfahren möglich, gezielt Polyglycidylverbindungen wirtschaftlich herzustellen und zu isolieren. Das erfindungsgemässe Verfahren kann als Kreisprozess durchgeführt werden, mit dem es möglich ist die nicht-epoxidierten und partiell epoxidierten Polyallylverbindungen, die stets zusammen mit den vollständig epoxidierten Polyallylverbindungen anfallen, abzutrennen und erneut mit Percarbonsäuren umzusetzen. Viele der erfindungsgemäss zugänglichen Polyglycidylverbindungen sind destillativ unter technischen Bedingungen aufgrund ihrer hohen Siedepunkte und ihrer Polymerisationsfreudigkeit nicht zu isolieren.

Nach dem erfindungsgemässen Verfahren ist es möglich, in einem für grosstechnische Anwendungen geeigneten, besonders wirtschaftlichen Verfahren mit einer Kombination von Destillationen und Extraktion unter schonenden Bedingungen die Polyglycidylverbindungen gezielt in hohen Ausbeuten herzustellen und zu isolieren.

In den folgenden Beispielen, die das erfindungsgemässe Verfahren weiter erläutern, aber nicht beschränken, bedeuten die Prozentangaben, soweit nichts anderes vermerkt ist, Gewichtsprozente.

Beispiele

In den Beispielen werden folgende Abkürzungen verwendet:
DA für Hexahydrophthalsäurediallylester
AG für Hexahydrophthalsäureallylglycidylester
DG für Hexahydrophthalsäurediglycidylester

Beispiel 1

In einem 60 l Rührreaktor mit Doppelmantel, innenliegenden Kühlschlangen und Rückflusskühler wurden 12,6 kg (50 Mol) DA vorgelegt und auf 60 °C erwärmt. Unter Rühren wurden 28,81 kg einer 20%igen Lösung von Perpropionsäure in Benzol (64 Mol) zugepumpt. Die Perpropionsäurelösung enthielt unter 0,1% Wasser, unter 0,2% Wasserstoffperoxid und unter 10 ppm Mineralsäure und wurde so zugegeben, dass die Temperatur bei 60 °C gehalten werden konnte. Danach wurde bei dieser Temperatur weiter gerührt. In zeitlichen Abständen wurden dem Reaktionsgemisch Proben entnommen und der zum jeweiligen Zeitpunkt noch vorhandene Gehalt an Perpropionsäure durch Titration und die Bildung von Epoxiden bzw. die Abnahme von epoxidierbaren Doppelbindungen gaschromatographisch durch Zuwiegen eines internen Standards bestimmt.

Nach einer Reaktionszeit von 5 Stunden betrug der Perpropionsäure-Umsatz 97,2% und der DA-Umsatz 84,8%. AG wurde mit einer Selektivität von 53,2% und DG mit einer Selektivität von 39,1%, jeweils bezogen auf umgesetzten DA gebildet.

Nach der Zugabe von 8,2 kg Dichlorbenzol wurde in einer mit einem Fallstromverdampfer betriebenen Füllkörperkolonne bei einem Druck von 150 mbar Benzol, Propionsäure und Dichlorbenzol als gemeinsames Kopfprodukt abgetrennt. Dieses wurde in einer weiteren Kolonne in die reinen Komponenten aufgetrennt. Als Sumpfprodukt hinterblieb ein Gemisch mit der Zusammensetzung 8% Dichlorbenzol, 13,0% DA, 41,1% AG und 32,1% DG. 4 g dieses Sumpfproduktes wurden mit 10 g eines Gemisches bestehend aus 70% Methanol und 30% Wasser (hydrophile Phase) und mit 20 g eines Gemisches bestehend aus 80% n-Heptan und 20% Benzol (lipophile Phase) in einem Scheidtrichter geschüttelt. Nach der Phasentrennung wurden beide Phasen gaschromatographisch analysiert, wobei sich folgende Gehalte ergaben:

| | hydrophile Phase | lipophile Phase |
|---|---|---|
| DA | 0,46% | 1,75% |
| AG | 0,57% | 4,22% |
| DG | 8,03% | 1,73% |

Damit ergibt sich für die Trennung von AG und DG folgender Trennfaktor B:

$$\beta = \frac{[AG]_{LP}}{[AG]_{HP}} \cdot \frac{[DG]_{HP}}{[DG]_{LP}} = 4{,}28$$

LP bedeutet dabei lipophile Phase;
HP bedeutet dabei hydrophile Phase;
die eckigen Klammern bedeuten die Konzentration der darin angegebenen Stoffe.

Die Isolierung von DG erfolgte mit Hilfe eines pulsierten Siebbodenextraktors, mit 70 praktischen Böden, durch Gegenstromextraktion. Dazu wurden am 20. Boden von oben gezählt stündlich 400 g des oben erhaltenen Sumpfproduktes in den Extraktor gepumpt. Am obersten Boden wurden stündlich 1000 g einer Mischung aus Methanol und Wasser (70:30) und am untersten Boden stündlich 2000 g einer Mischung aus Heptan und Benzol (80:20) zugepumpt. Am Sumpf wurde die Methanol/Wasser-Phase und am Kopf die Heptan/Benzol-Phase abgenommen. Diese wiesen folgende Gehalte auf:

| | Extraktor-Kopfprodukt (Heptan/Benzol-Phase) | Extraktor-Sumpfprodukt (Methanol/Wasser-Phase) |
|---|---|---|
| DA | 2,32% | – |
| AG | 7,26% | 0,07% |
| DG | 1,11% | 9,37% |

Aus dem Sumpfprodukt des Extraktors wurden in einem Dünnschichtverdampfer bei 150 mbar Methanol und Wasser als gemeinsames Kopfprodukt abdestilliert und in den Extraktor zurückgeführt. Es hinterblieb DG in Form eines hellgelben Öles, das weniger als 1% AG enthielt.

Aus dem Kopfprodukt des Extraktors wurden in einem Fallstromverdampfer bei 150 mbar Heptan und Benzol als gemeinsames Kopfprodukt abdestilliert und in den Extraktor zurückgeführt. Als Sumpfprodukt hinterblieb ein Gemisch, das DA, AG und DG enthielt. Dieses wurde, nach Zusatz von frischem DA, erneut zur Umsetzung mit Perpropionsäurelösung zurückgeführt.

Die in den Extraktor zurückgeführten Lösungsmittelgemische hatten folgende Zusammensetzung:

| | Heptan-Benzol-Gemisch | Methanol-Wasser-Gemisch |
|---|---|---|
| Heptan | 77,7 % | 0,7% |
| Benzol | 19,6 % | 2,3% |
| Methanol | 2,55% | 67,0% |
| Wasser | 0,15% | 30,0% |

Beispiel 2

In analoger Weise wie in Beispiel 1 beschrieben wurde durch Umsetzung von 12,6 kg (50 Mol) DA mit 31,5 kg 20%-iger benzolischer Perpropionsäurelösung (= 70 Mol Perpropionsäure) bei 60 °C während 6 Stunden und anschliessender, nach Zugabe von Dichlorbenzol durchgeführter Destillation des Reaktionsgemisches, ein Gemisch erhalten, das 11,4% DA, 44,9% AG und 35,8% DG enthielt.

4 g dieses Gemisches wurden in einem Scheidetrichter mit 12 g eines Gemisches aus 70% Methanol und 30% Wasser (hydrophile Phase) und 24 g Cyclohexan (lipophile Phase) geschüttelt. Nach der Phasentrennung wurden beide Phasen gaschromatographisch analysiert, wobei sich folgende Gehalte ergaben:

| | hydrophile Phase | lipophile Phase |
|---|---|---|
| DA | 0,28% | 1,79% |
| AG | 3,47% | 4,21% |
| DG | 5,05% | 1,27% |

Damit ergibt sich für die Trennung von AG und DG ein Trennfaktor von β = 4,8.

Bei der extraktiven Aufarbeitung des Gemisches, die entsprechend Beispiel 1 mit 400 g/h DA/AG/DG-Gemisch, 1200 g/h Methanol/Wasser-Gemisch (70:30) und 2400 g/h Cyclohexan durchgeführt wurde, wurden 81% des im Gemisch vorhandenen DG abgetrennt. Der abgetrennte DG enthielt 1% AG.

Beispiel 3

4 g des gleichen Gemisches wie in Beispiel 2 wurden in 10 g Methanol/Wasser (70:30) gelöst und mit 20 g einer Mischung bestehend aus 80% Heptan und 20% Tetrahydrofuran im Scheidetrichter geschüttelt. Nach der Phasentrennung wurden beide Phasen gaschromatographisch analysiert, wobei sich der Trennfaktor zu β = 4,9 ergab.

Bei der extraktiven Aufarbeitung des Gemisches, die entsprechend Beispiel 1 mit 400 g/h DA/AG/DG-Gemisch, 1000 g/h Methanol/Wasser-Gemisch (70:30) und 2000 g/h Heptan/Tetrahydrofuran-Gemisch (80:20) durchgeführt wurde, wurden 79% des im Gemisch vorhandenen DG abgetrennt. Der abgetrennte DG enthielt 0,9% AG.

Aus der beladenen lipophilen Phase wurde die unbeladene liphophile Phase durch Destillation zurückgewonnen und in den Extraktor zurückgeführt. Das Sumpfprodukt dieser Destillation wurde nach Zugabe von frischem DA in den Reaktor zurückgeführt.

Beispiel 4

4 g des gleichen Gemisches wie in Beispiel 2 wurde in einem Scheidetrichter mit 20 g einer Mischung aus Heptan/Benzol (80:20) als lipophile Phase und mit 10 g einer Mischung aus Diethylenglykol/Wasser (80:20) als hydrophile Phase geschüttelt. Nach der Phasentrennung wurden beide Phasen gaschromatographisch analysiert. Es ergab sich für die Trennung AG/DG ein Trennfaktor von β = 5,1.

Bei der extraktiven Aufarbeitung des Gemisches, die entsprechend Beispiel 1 mit 400 g/h DA/AG/DG-Gemisch, 2000 g/h Heptan/Benzol-Gemisch (80:20) und 1000 g/h Diethylenglykol/Wasser-Gemisch (80:20) durchgeführt wurde, wurden 78% des im Gemisch vorhandenen DG abgetrennt. Der abgetrennte DG enthielt 0,8% AG.

Beispiel 5

10 g des gleichen Gemisches wie in Beispiel 2 wurden in einem Scheidetrichter mit 5 g eines Gemisches, bestehend aus N-Methylpyrrolidon und Wasser (70:30) als hydrophile Phase und 15 g Cyclohexan als lipophile Phase geschüttelt. Nach der Phasentrennung wurden beide Phasen gaschromatographisch analysiert, wobei sich für die Trennung AG/DG ein Trennfaktor von β = 4,6 ergab.

Bei der extraktiven Aufarbeitung des Gemisches, die entsprechend Beispiel 1 mit 1000 g/h DA/AG/DG-Gemisch, 500 g/h N-Methylpyrrolidon/Wasser-Gemisch (70:30) und 1500 g/h Cyclohexan durchgeführt wurde, wurden 77,5% des im Gemisch vorhandenen DG abgetrennt. Der abgetrennte DG enthielt 0,7% AG.

Beispiel 6

10 g des gleichen Gemisches wie in Beispiel 2 wurden in einem Scheidetrichter mit 5 g eines Gemisches bestehend aus Sulfolan und Wasser (80:20) als hydrophiler Phase und 15 g Cyclohexan als lipophiler Phase geschüttelt. Nach der Phasentrennung wurden beide Phasen gaschromatographisch analysiert, wobei sich für die Trennung von AG von DG ein Trennfaktor β von 5,3 ergab.

Bei der extraktiven Aufarbeitung des Gemisches, die entsprechend Beispiel 1 mit 1000 g/h DA/AG/DG-Gemisch, 500 g/h Sulfolan/Wasser-Gemisch (80:20) und 1500 g/h Cyclohexan durchgeführt wurde, wurden 80,5% des im Gemisch vorhandenen DG abgetrennt. Der abgetrennte DG enthielt 1% AG.

Beispiel 7

12,3 kg (50 Mol) Phthalsäurediallylester wurden wie in Beispiel 1 beschrieben mit 27,042 kg einer 21,3%igen Lösung von Perpropionsäure in Benzol (64 Mol) bei 60 °C umgesetzt. Nach einer Reaktionszeit von 7 Stunden resultierte ein Persäureumsatz von 96,9%. Der Umsatz an Diallylphthalat betrug 85,5%. Allylglycidylphthalat wurde mit einer Selektivität von 51,8% und Diglycidylphthalat mit einer Selektivität von 42,3%, jeweils bezogen auf umgesetzten Diallylester, gebildet.

Nach Zugabe von 8 kg Dichlorbenzol wurde wie im Beispiel 1 beschrieben aufgearbeitet. Es resultierte nach Abtrennen von Benzol und Propionsäure ein Gemisch der Zusammensetzung: 6% Dichlorbenzol, 13,1% Phthalsäurediallylester, 41,2% Phthalsäureallylglycidylester und 34,8% Phthalsäurediglycidylester.

4 g dieses Gemisches wurden in einem Scheidetrichter mit 10 g eines Gemisches bestehend aus 65% Methanol und 35% Wasser (hydrophile Phase) und 20 g Cyclohexan (lipophile Phase) ausgeschüttelt. Nach der Phasentrennung wurden beide Phasen gaschromatographisch analysiert, wobei sich für die Trennung von Phthalsäureallylglycidylester von Phthalsäurediglycidylester ein Trennfaktor von β = 4,8 ergab.

Das Dichlorbenzol, Phthalsäurediallylester, Phthalsäureallylglycidylester und Phthalsäurediglycidylester enthaltende Gemisch wurde in entsprechender Weise wie im Beispiel 1 beschrieben durch Extraktion getrennt. Dabei wurden 400 g/h des Estergemisches, 1000 g/h Methanol/Wasser-Gemisch (65:35) und 2000 g/h Cyclohexan eingesetzt. Aus der Methanol/Wasser-Phase wurden 78% des im Estergemisch enthaltenen Phthalsäurediglycidylesters abgetrennt, mit einem Gehalt an Phthalsäureallylglycidylester von weniger als 1%. Aus der Cyclohexan-Phase wurde ein Gemisch der Zusammensetzung 5,4% Dichlorbenzol, 19,5% Diallylphthalat, 61,4% Allylglycidylphthalat und 11,4% Diglycidylphthalat abgetrennt, was nach Zusatz von frischem Phthalsäurediallylester erneut der Umsetzung mit benzolischer Perpropionsäurelösung zugeführt wurde.

Beispiel 8

12,6 kg (50 Mol) Hexahydroterephthalsäurediallylester wurden, entsprechend der in Beispiel 1 beschriebenen Arbeitsweise, mit 26,79 kg einer 21,5%-igen Lösung von Perpropionsäure in Benzol (64 Mol) bei 60 °C umgesetzt. Nach einer Reaktionszeit von 7 Stunden resultierte ein Persäureumsatz von 97,1%. Der Umsatz an Hexahydroterephthalsäurediallylester betrug 85%. Hexahydroterephthalsäureallylglycidylester wurde mit einer Selektivität von 52,8% und Hexahydrophthalsäurediglycidylester mit einer Selektivität von 39,6%, jeweils bezogen umgesetzten Hexahydroterephthalsäurediallylester gebildet.

Nach Zugabe von 8,5 kg Dichlorbenzol wurden in entsprechender Weise, wie in Beispiel 1 beschrieben, Benzol, Propionsäure und Dichlorbenzol als gemeinsames Kopfprodukt abdestilliert. Dabei wurde ein Sumpfprodukt erhalten, das 6,4% Dichlorbenzol, 13,0% Hexahydroterephthalsäurediallylester, 42,2% Hexahydroterephthalsäureallylglycidylester und 32,2% Hexahydroterephthalsäurediglycidylester enthielt. 10 g dieses Gemisches wurden in einem Scheidetrichter mit 15 g eines Gemisches aus 70% N-Methylpyrrolidon und 30% Wasser (hydrophile Phase) und mit 30 g eines Gemisches aus 60% Cyclohexan und 40% Dichlorbenzol (lipophile Phase) geschüttelt. Nach der Phasentrennung wurden die beiden Phasen gaschromatographisch analysiert, wobei sich für die Trennung von Hexahydroterephthalsäureallylglycidylester von Hexahydroterephthalsäurediglycidylester ein Trennfaktor von $\beta = 5{,}3$ ergab.

Die extraktive Trennung des Estergemisches erfolgte in entsprechender Weise wie in Beispiel 1 beschrieben. Dabei wurden stündlich 1 kg des Estergemisches, 3 kg eines Gemisches aus 60% Cyclohexan und 40% Dichlorbenzol und 1,5 kg eines Gemisches aus 70% N-Methylpyrrolidon und 30% Wasser in den Extraktor gepumpt.

Aus der hydrophilen Phase wurden 85% des im Estergemisch enthaltenen Hexahydroterephthalsäurediglycidylesters abgetrennt. Dieser enthielt 1,5% Hexahydroterephthalsäureallylglycidylester.

Beispiel 9

9,9 kg (50 Mol) Bernsteinsäurediallylester wurden mit 28,9 kg einer 21,8%igen Lösung von Perpropionsäure in Benzol (70 Mol) in entsprechender Weise wie in Beispiel 1 beschrieben bei 60 °C umgesetzt. Nach einer Reaktionszeit von 8 Stunden resultierten ein Persäureumsatz von 97,0% und ein Umsatz an Bernsteinsäurediallylester von 88%. Bernsteinsäureallylglycidylester wurde mit einer Selektivität von 46,3% Bernsteinsäurediglycidylester mit einer Selektivität von 46,1%, jeweils bezogen auf umgesetzten Bernsteinsäurediallylester, gebildet.

Nach Zugabe von 6 kg Dichlorbenzol wurden, entsprechend der Arbeitsweise wie in Beispiel 1 beschrieben, Benzol und Propionsäure zusammen mit Dichlorbenzol abdestilliert. Es hinterblieb ein Gemisch mit der Zusammensetzung 7,8% Dichlorbenzol, 10,0% Bernsteinsäurediallylester, 37,7% Bernsteinsäureallylglycidylester und 37,3% Bernsteinsäurediglycidylester.

10 g dieses Estergemisches wurden mit 20 g einer Mischung aus 55% Methanol und 45% Wasser (hydrophile Phase) und 40 g einer Mischung aus 75% Cyclohexan und 25% Benzol (lipophile Phase) in einem Scheidetrichter geschüttelt. Nach der Phasentrennung wurden die beiden Phasen gaschromatographisch analysiert, wobei sich für die Trennung von Bernsteinsäureallylglycidylester von Bernsteinsäurediglycidylester ein Trennfaktor $\beta$ von 6,8 ergab.

Die extraktive Aufarbeitung des Estergemisches erfolgte in entsprechender Weise, wie in Beispiel 1 beschrieben. Dabei wurden dem Extraktor stündlich 600 g des Estergemisches, 1,2 kg Methanol/Wasser-Gemisch (55:45) und 2,4 kg Cyclohexan/Benzol-Gemisch (75:25) zugeführt. Aus der hydrophilen Phase wurden 78% des im Estergemisch enthaltenen Bernsteinsäurediglycidylesters isoliert. Dieser enthielt 0,9% Bernsteinsäureallylglycidylester.

Beispiel 10

9,8 kg (50 Mol) Maleinsäurediallylester wurden mit 28,9 kg einer 21,8%igen Lösung von Perpropionsäure in Benzol (70 Mol) in analoger Weise wie in Beispiel 1 beschrieben bei einer Reaktionstemperatur von 60 °C umgesetzt. Nach einer Reaktionszeit von 8,5 Stunden betrug der Percarbonsäureumsatz 97,2%. Der Umsatz an Maleinsäurediallylester betrug 88,3%. Maleinsäureallylglycidylester wurde mit einer Selektivität von 45,8% und Maleinsäurediglycidylester mit einer Selektivität von 44,7%, jeweils bezogen auf umgesetzten Maleinsäurediallylester, gebildet.

Nach Zugabe von 6 kg Dichlorbenzol wurden, in analoger Weise wie im Beispiel 1 beschrieben, Benzol, Propionsäure und Dichlorbenzol als gemeinsames Kopfprodukt abdestilliert. Im Sumpf hinterblieb ein Gemisch der Zusammensetzung 6,5% Dichlorbenzol, 10,3% Maleinsäurediallylester, 37,3% Maleinsäureallylglycidylester und 37,0% Maleinsäurediglycidylester.

10 g dieses Estergemisches wurden mit 20 g einer Mischung bestehend aus 57,5% Methanol und 42,5% Wasser (hydrophile Phase) und mit 40 g einer Mischung bestehend aus 70% Cyclohexan und 30% Dichlorbenzol (lipophile Phase) im Scheidetrichter ausgeschüttelt. Nach der Phasentrennung wurden beide Phasen gaschromatographisch analysiert, wobei sich für die Trennung von Maleinsäureallylglycidylester von Maleinsäurediglycidylester ein Trennfaktor von $\beta = 5{,}6$ ergab.

Die extraktive Aufarbeitung des Estergemisches erfolgte in analoger Weise wie in Beispiel 1 beschrieben. Dem Extraktor wurden stündlich 600 g Estergemisch, 1,2 kg einer Mischung aus 57,5% Methanol und 42,5% Wasser und 2,4 kg einer Mischung aus 70% Cyclohexan und 30% Dichlorbenzol zugeführt. Aus der Methanol/

Wasser-Phase wurden 82% des im Estergemisch enthaltenen Maleinsäurediglycidylester isoliert. Dieser enthielt weniger als 0,8% Maleinsäureallylglycidylester.

Beispiel 11

9,8 kg (50 Mol) Fumarsäurediallylester wurden mit 26,18 kg einer 22%-igen Lösung von Perpropionsäure in Benzol (64 Mol) bei 60 °C in analoger Weise wie in Beispiel 1 beschrieben umgesetzt. Nach einer Reaktionsdauer von 6,8 Stunden resultierte ein Percarbonsäureumsatz von 96,8%. Der Umsatz an Fumarsäurediallylester betrug 90%. Fumarsäureallylglycidylester wurde mit einer Selektivität von 46,2% und Fumarsäurediglycidylester mit einer Selektivität von 42,3%, jeweils bezogen auf umgesetzten Fumarsäurediallylester, gebildet.

Nach Zugabe von 12 kg Dichlorbenzol wurden in analoger Weise wie in Beispiel 1 beschrieben Benzol, Propionsäure und Dichlorbenzol als gemeinsames Kopfprodukt abdestilliert. Es hinterblieb ein Gemisch der Zusammensetzung 34% Dichlorbenzol, 6,5% Fumarsäurediallylester, 26,2% Fumarsäureallylglycidylester und 23,9% Fumarsäurediglycidylester.

10 g dieses Estergemisches wurden mit 24 g einer Mischung bestehend aus 75% N-Methylpyrrolidon und 25% Wasser (hydrophile Phase) und mit 22 g einer Mischung bestehend aus 65% Cyclohexan und 35% Dichlorbenzol (lipophile Phase) in einem Scheidetrichter ausgeschüttelt. Nach der Phasentrennung wurden beide Phasen gaschromatographisch analysiert, wobei sich für die Trennung von Fumarsäureallylglycidylester von Fumarsäurediglycidylester ein Trennfaktor von $\beta = 4{,}2$ ergab.

Die extraktive Aufarbeitung des Estergemisches wurde in entsprechender Weise, wie in Beispiel 1 beschrieben, vorgenommen, wobei dem Extraktor stündlich 600 g des Estergemisches, 1,5 kg einer Mischung aus N-Methylpyrrolidon und Wasser (75:25) und 2,5 kg einer Mischung aus Cyclohexan und Dichlorbenzol (65:35) zugeführt wurde.

Aus der N-Methylpyrrolidon/Wasser-Phase wurden 82% des im Estergemisch enthaltenen Fumarsäurediglycidylesters als kristallines Produkt erhalten, das weniger als 1% Fumarsäureallylglycidylester enthielt.

Beispiel 12

6,895 kg (35 Mol) Trisallylurazol wurden in analoger Weise wie in Beispiel 1 beschrieben mit 31,5 kg einer 22%-igen Lösung von Perpropionsäure in Benzol (77 Mol) bei 60 °C umgesetzt. Nach einer Reaktionszeit von 10 Stunden betrugen der Percarbonsäureumsatz 97,2% und der Umsatz an Trisallylurazol 94,6%. Diallylglycidylurazol wurde in einer Selektivität von 28,3%, Allyldiglycidylurazol in einer Selektivität von 41,2% und Trisglycidylurazol mit einer Selektivität von 20,1%, jeweils bezogen auf umgesetztes Trisallylurazol, gebildet.

Nach Zugabe von 8 kg Dichlorbenzol wurden, entsprechend Beispiel 1, Benzol, Propionsäure und Dichlorbenzol als gemeinsames Kopfprodukt abdestilliert. Als Sumpfprodukt resultierte ein Gemisch der Zusammensetzung 50% Dichlorbenzol, 2,3% Trisallylurazol, 12,4% Diallylglycidylurazol, 20,0% Allyldiglycidylurazol und 10,1% Trisglycidylurazol.

10 g dieses Gemisches wurden mit 7,5 g eines Gemisches bestehend aus 20% Methanol und 80% Wasser (hydrophile Phase) und mit 12,5 g Dichlorbenzol (lipophile Phase) in einem Scheidetrichter geschüttelt. Nach der Phasentrennung wurden die beiden Phasen gaschromatographisch analysiert, wobei sich für die Trennung von Allyldiglycidylurazol von Trisglycidylurazol ein Trennfaktor von $\beta = 3{,}6$ ergab.

Die extraktive Aufarbeitung des Gemisches erfolgte in analoger Weise wie in Beispiel 1 beschrieben, wobei dem Extraktor stündlich im oberen Drittel 1 kg des Gemisches, am obersten Boden 0,75 kg eines Gemisches aus 20% Methanol und 80% Wasser und am untersten Boden 1,25 kg Dichlorbenzol zugeführt wurden. Aus der Methanol/Wasser-Phase wurden 72% des im Gemisch enthaltenen Trisglycidylurazols isoliert, das noch 4,8% Allyldiglycidylurazol enthielt.

**Patentansprüche**

1. Verfahren zur Herstellung von Polyglycidylverbindungen, indem man eine Polyallylverbindung der allgemeinen Formel

$$R-(\overset{\overset{O}{\|}}{C}-O-CH_2-CH=CH_2)_n$$

worin

R für einen, gegebenenfalls substituierten, gesättigten, aliphatischen Rest mit 1 bis 10 C-Atomen, für die Gruppe $-CH=CH-$, einen, gegebenenfalls substituierten, gesättigten oder ungesättigten cycloaliphatischen Rest mit 5 bis 12 C-Atomen, für einen, gegebenenfalls substituierten, Benzolrest oder für einen, gegebenenfalls substituierten, Naphthalinrest und

n für 2, 3 oder 4 steht

oder eine Triallylverbindung von Cyanursäure, Isocyanursäure oder Urazol bei 30 bis 100 °C mit 0,6 bis 1,1 Mol einer 3 oder 4 Kohlenstoffatome enthaltenden Percarbonsäure, bezogen auf ein Mol zu epoxidierender Doppelbindung, umsetzt, anschliessend durch Destillation die aus der Percarbonsäure entstandene Carbonsäure abtrennt, dadurch gekennzeichnet, dass man das verbleibende Gemisch durch gleichzeitige Extraktion mit einer lipophilen und einer hydrophilen Phase auftrennt, anschliessend getrennt die beladene lipophile und die beladene hydrophile Phase in an sich bekannter Weise aufarbeitet, und als Rückstand aus der Aufarbeitung der beladenen hydrophilen Phase die Polyglycidylverbindung erhält.

2. Verfahren nach Anspruch 1, dadurch gekennzeichnet, dass man als Polyallylverbindungen die Polyallylester von

Hexahydrophthalsäure, Hexahydroisophthalsäure,
Hexahydroterephthalsäure,
3-Methylhexahydrophthalsäure,
3,5-Dimethylhexahydrophthalsäure,
hydrierten Benzoltri- und -tetracarbonsäuren,
Tetrahydrophthalsäure, 3-Methylphthalsäure,
3,5-Dimethylphthalsäure, 3-Methoxyphthalsäure,
Terephthalsäure, Benzoltricarbonsäuren,
Benzoltetracarbonsäuren,
Naphthalindicarbonsäuren, Malonsäure,
Bernsteinsäure, Glutarsäure, Adipinsäure,
Pimelinsäure, Suberinsäure, Decandicarbonsäure,
Maleinsäure oder Fumarsäure
oder die Trisallylverbindungen von Cyanursäure, Isocyanursäure oder Urazol einsetzt.

3. Verfahren nach Ansprüchen 1 bis 2, dadurch gekennzeichnet, dass man die Percarbonsäure in Form einer Lösung in einem organischen Lösungsmittel einsetzt, die weniger als 10 Gew.-% Wasser, weniger als 1 Gew.-% Wasserstoffperoxid und weniger als 50 ppm Mineralsäure enthält.

4. Verfahren nach Ansprüchen 1 bis 3, dadurch gekennzeichnet, dass man die Destillation zur Abtrennung der Carbonsäure in Gegenwart eines Lösungsmittels durchführt, das zwischen der Carbonsäure und der Polyallylverbindung siedet.

5. Verfahren nach Ansprüchen 1 bis 4, dadurch gekennzeichnet, dass man als lipophile Phase Cycloalkane oder Mischungen aus 60 bis 90 Gew.-% Alkanen mit 5 bis 12 C-Atomen oder 60 bis 90 Gew.-% Cycloalkanen und jeweils 10 bis 40 Gew.-% Benzol, Toluol, Xylol, Chlorbenzol, Dichlorbenzol, Chloroform, Butylacetat oder Tetrahydrofuran verwendet.

6. Verfahren nach Ansprüchen 1 bis 5, dadurch gekennzeichnet, dass man als hydrophile Phase Ethylenglykol, Diethylenglykol, Sulfolan oder N-Methylpyrrolidon oder Mischungen aus 30 bis 80 Gew.-% Wasser und 20 bis 70 Gew.-% Methanol, Ethanol oder Isopropanol oder Mischungen aus 10 bis 25 Gew.-% Wasser und 75 bis 90 Gew.-% Ethylenglykol, Diethylenglykol oder Sulfolan oder Mischungen aus 10 bis 80 Gew.-% Wasser und 20 bis 90 Gew.-% N-Methylpyrrolidon oder Mischungen aus 50 bis 90 Gew.-% Ethylenglykol mit 10 bis 50 Gew.-% Methanol, Ethanol oder Isopropanol verwendet.

7. Verfahren nach Ansprüchen 1 bis 6, dadurch gekennzeichnet, dass das Gewichtsverhältnis von lipophiler zu hydrophiler Phase 1:1 bis 5:1 beträgt.

8. Verfahren nach Ansprüchen 1 bis 9, dadurch gekennzeichnet, dass man die Extraktion bei Temperaturen im Bereich 10 bis 60 °C durchführt.

9. Verfahren nach Ansprüchen 1 bis 8, dadurch gekennzeichnet, dass man in Fällen, in denen nach der Destillation des Reaktionsgemisches zur Abtrennung der Carbonsäure ein fester Rückstand anfällt, frische Polyallylverbindung dem Sumpfprodukt dieser Destillation zufügt und das Sumpfprodukt der Destillation zusammen mit der frischen Polyallylverbindung der Extraktion zuführt.

**Claims**

1. A process for the production of polyglycidyl compounds in which a polyallyl compound corresponding to the following general formula

$$R-(\overset{O}{\overset{\|}{C}}-O-CH_2-CH=CH_2)_n$$

in which

R represents an optionally substituted, saturated aliphatic $C_1$–$C_{10}$ radical, the group $-CH=CH-$, an optionally substituted, saturated or unsaturated cycloaliphatic $C_5$–$C_{12}$ radical, an optionally substituted benzene radical or an optionally substituted naphthalene radical and

n = 2, 3 or 4,

or a triallyl compound of cyanuric acid, isocyanuric acid or urazole is reacted at 30 to 100 °C with from 0.6 to 1.1 mole of a $C_3$ or $C_4$ percarboxylic acid per mole of double bond to be epoxidized and the carboxylic acid formed from the percarboxylic acid is separated off by distillation, characterized in that the residual mixture is separated up by simultaneous extraction with a lipophilic and a hydrophilic phase, the charged lipophilic phase and the charged hydrophilic phase are then separately worked up in known manner and the polyglycidyl compound is obtained as residue from the working up of the charged hydrophilic phase.

2. A process as claimed in Claim 1, characterized in that the polyallyl esters of hexahydrophthalic acid, hexahydroisophthalic acid, hexahydroterephthalic acid, 3-methylhexahydrophthalic acid, 3,5-dimethylhexahydrophthalic acid, hydrogenated benzene tri- and tetracarboxylic acids, tetrahydrophthalic acid, 3-methylphthalic acid, 3,5-dimethylphthalic acid, 3-methoxyphthalic acid, terephthalic acid, benzene tricarboxylic acids, benzene tetracarboxylic acids, naphthalene dicarboxylic acids, malonic acid, succinic acid, glutaric acid, adipic acid, pimelic acid, suberic acid, decane dicarboxylic acid, maleic acid or fumaric acid or the triallyl compounds of cyanuric acid, isocyanuric acid or urazole are used as the polyallyl compounds.

3. A process as claimed in Claims 1 and 2, characterized in that the percarboxylic acid is used in the form of a solution in an organic solvent which contains less than 10% by weight water, less than 1% by weight hydrogen peroxide and less than 50 ppm mineral acid.

4. A process as claimed in Claims 1 to 3, characterized in that the distillation to remove the carboxylic acid is carried out in the presence of a solvent which boils between the carboxylic acid and the polyallyl compound.

5. A process as claimed in Claims 1 to 4, characterized in that cycloalkanes or mixtures of from 60 to 90% by weight $C_5$–$C_{12}$ alkanes or from 60 to 90% by weight cycloalkanes and from 10 to 40% by weight benzene, toluene, xylene, chlorobenzene, dichlorobenzene, chloroform, butylacetate or tetrahydrofuran are used as the lipophilic phase.

6. A process as claimed in Claims 1 to 5, characterized in that ethylene glycol, diethylene glycol, sulfolan or N-methyl pyrrolidone or mixtures of from 30 to 80% by weight water and from 20 to 70% weight methanol, ethanol or isopropanol or mixtures of from 10 to 25% by weight water and from 75 to 90% by weight ethylene glycol, diethylene glycol or sulfolan or mixtures of from 10 to 80% by weight water and from 20 to 90% weight N-methyl pyrrolidone or mixtures of from 50 to 90% by weight ethylene glycol with from 10 to 50% by weight methanol, ethanol or isopropanol are used as the hydrophilic phase.

7. A process as claimed in Claims 1 to 6, characterized in that the ratio by weight of lipophilic to hydrophilic phase is from 1:1 to 5:1.

8. A process as claimed in Claims 1 to 9, characterized in that the extraction is carried out at temperatures of from 10 to 60 °C.

9. A process as claimed in Claims 1 to 8, characterized in that, in cases where a solid residue is obtained after distillation of the reaction mixture to remove the carboxylic acid, fresh polyallyl compound is added to the sump product of this distillation and the sump product of the distillation is fed with the fresh polyallyl compound to the extraction stage.

**Revendications**

1. Procédé de production de composés polyglycidyliques, dans lequel on fait réagir un composé polyallylique de formule générale

$$R{-}(\overset{O}{\overset{\|}{C}}{-}O{-}CH_2{-}CH{=}CH_2)_n$$

dans laquelle

R représente un reste aliphatique saturé de 1 à 10 atomes de carbone, éventuellement substitué, le groupe $-CH=CH-$, un reste cycloaliphatique de 5 à 12 atomes de carbone saturé ou non saturé, éventuellement substitué, un reste benzénique éventuellement substitué ou un reste naphtalénique éventuellement substitué et

n a la valeur 2, 3 ou 4,

ou bien un composé triallylique de l'acide cyanurique, de l'acide isocyanurique ou de l'urazole à une température de 30 à 100 °C avec 0,6 à 1,1 mole d'un acide percarboxylique contenant 3 ou 4 atomes de carbone, par rapport à 1 mole de double liaison à époxyder, puis on sépare par distillation l'acide carboxylique produit à partir de l'acide percarboxylique, caractérisé en ce qu'on divise le mélange restant par extraction simultanée avec une phase lipophile et une phase hydrophile, puis on traite séparément d'une manière connue la phase lipophile chargée et la phase hydrophile chargée et on obtient le composé polyglycidylique comme résidu du traitement de la phase hydrophile chargée.

2. Procédé suivant la revendication 1, caractérisé en ce qu'on utilise comme composés polyallyliques les esters polyallyliques de l'acide hexahydrophtalique, de l'acide hexahydroisophtalique, de l'acide hexahydrotéréphtalique, de l'acide 3-méthylhexahydrophtalique, de l'acide 3,5-diméthylhexahydrophtalique, d'acides benzènetricarboxyliques et tétracarboxyliques hydrogénés, de l'acide tétrahydrophtalique, de l'acide 3-méthylphtalique, de l'acide 3,5-diméthylphtalique, de l'acide 3-méthoxyphtalique, de l'acide téréphtalique, des acides benzènetricarboxyliques, des acides benzènetétracarboxyliques, des acides naphtalènedicarboxyliques, de l'acide malonique, de l'acide succinique, de l'acide glutarique, de l'acide adipique, de l'acide pimélique, de l'acide subérique, de l'acide décanedicarboxylique, de l'acide maléique ou de l'acide fumarique ou les composés trisallyliques de l'acide cyanurique, de l'acide isocyanurique ou de l'urazole.

3. Procédé suivant les revendications 1 et 2, caractérisé en ce qu'on utilise l'acide percarboxylique sous la forme d'une solution dans un solvant organique qui contient moins de 10% en poids d'eau, moins de 1% en poids de peroxyde d'hydrogène et moins de 50 ppm d'acide minéral.

4. Procédé suivant les revendications 1 à 3, caractérisé en ce qu'on conduit la distillation en vue de la séparation de l'acide carboxylique en présence d'un solvant dont le point d'ébullition se situe entre ceux de l'acide carboxylique et du composé polyallylique.

5. Procédé suivant les revendications 1 à 4, caractérisé en ce qu'on utilise comme phase lipophile des cyclo-alcanes ou des mélanges de 60 à 90% en poids d'alcanes ayant 5 à 12 atomes de carbone ou de 60 à 90% en poids de cyclo-alcanes et, dans chaque cas, 10 à 40% en poids de benzène, de toluène, de xylène, de chlorobenzène, de dichlorobenzène, de chloroforme, d'acétate de butyle ou de tétrahydrofuranne.

6. Procédè suivant les revendications 1 à 5, caractérisé en ce qu'on utilise comme phase hydrophile l'éthylène-glycol, le diéthylène-glycol, le sulfolane ou la N-méthylpyrrolidone ou des mélanges de 30 à 80% en poids d'eau et de 20 à 70% en poids de méthanol, d'éthanol ou d'isopropanol ou des mélanges de 10 à 25% en poids d'eau et de 75 à 90% en poids d'éthylèneglycol, de diéthylène-glycol ou de sulfolane ou des mélanges de 10 à 80% en poids d'eau et de 20 à 90% en poids de N-méthylpyrrolidone ou des mélanges de 50 à 90% en poids d'éthylène-glycol avec 10 à 50% en poids de méthanol, d'éthanol ou d'isopropanol.

7. Procédé suivant les revendications 1 à 6, caractérisé en ce que le rapport en poids de la phase lipophile à la phase hydrophile va de 1:1 à 5:1.

8. Procédé suivant les revendications 1 à 9, caractérisé en ce qu'on conduit l'extraction à des températures comprises dans l'intervalle de 10 à 60 °C.

9. Procédé suivant les revendications 1 à 8, caractérisé en ce que, dans les cas où un résidu solide reste après la distillation du mélange réactionnel en vue de la séparation de l'acide carboxylique, on ajoute au produit de queue de cette distillation du composé polyallylique frais et on soumet le produit de queue à l'extraction conjointement avec le composé polyallylique frais.